# EUROPEAN PATENT APPLICATION

(11) **EP 2 843 043 A1**
(43) Date of publication of application: **04.03.2015**
(21) Application number: 13181841.1
(22) Date of filing: 27.08.2013
(51) Int. Cl.: C12N 9/10, C12N 9/00, A61K 8/44, C12P 13/04

(54) **A method for producing acyl amino acids**

(71) Applicant: Evonik Industries AG, 45128 Essen (DE)
(72) Inventor: Grammann, Katrin, 45739 Oer-Erkenschwick (DE); Wolter, Jan, 40489 Düsseldorf (DE); Schaffer, Steffen, 45699 Herten (DE); Gielen, Jasmin, 44879 Bochum (DE); Haas, Thomas, 48161 Münster (DE); Potgrave, Nicole, 46286 Dorsten (DE)

(57) **Abstract**

The present invention relates to a cell expressing an amino acid-N-acyl-transferase, which is preferably recombinant, and an acyl-CoA synthetase, which is preferably recombinant, wherein the cell has a reduced fatty acid degradation capacity, a method for producing acyl amino acids, comprising the step contacting an amino acid and an acyl CoA in the presence of an amino acid-N-acyl-transferase, which is preferably isolated and/or recombinant, wherein the amino acid-N-acyl-transferase is preferably a human amino acid-N-acyl-transferase, or culturing the cell and a reaction mixture comprising an amino acid-N-acyl-transferase, which is preferably isolated and/or recombinant, an acyl-CoA synthetase, which is preferably isolated and/or recombinant, an amino acid and either a fatty acid-CoA or a fatty acid and an acyl-CoA-synthase.

## Description

The present invention relates to a cell expressing an amino acid-N-acyl-transferase, which is preferably recombinant, and an acyl-CoA synthetase, which is preferably recombinant, wherein the cell has a reduced fatty acid degradation capacity, a method for producing acyl amino acids, comprising the step contacting an amino acid and fatty acid-CoA in the presence of an amino acid-N-acyl-transferase, which is preferably isolated and/or recombinant, wherein the amino acid-N-acyl-transferase is preferably a human amino acid-N-acyl-transferase, or culturing the cell, and a reaction mixture comprising an amino acid-N-acyl-transferase, which is preferably isolated and/or recombinant, an acyl-CoA synthetase, which is preferably isolated and/or recombinant, an amino acid and either a fatty acid-CoA or a fatty acid and an acyl-CoA-synthase.

Acyl amino acids are a class of surface-active agents with a variety of uses, for example as detergents for washing purposes, emulsifiers in food products and as essential ingredients in various personal care products such as shampoos, soaps, moisturizing agents and the like. In addition to having both hydrophobic and hydrophilic regions, a prerequisite for use as a surfactant, the compounds are made of naturally occurring molecules, more specifically amino acids and fatty acids, which are not only non-hazardous and environmentally acceptable but may be readily produced at a large scale using inexpensive biological raw materials. In pharmacological research, acyl amino acids are used as neuromodulators and probes for new drug targets.

Acyl amino acids have been isolated from a multitude of biological sources and are believed to have a range of functions, for example as signaling molecules in mammalian tissues (Tan, B., O'Dell, D. K., Yu, Y. W., Monn, M. F., Hughes, H. V., Burstein, S., Walker, J.M. (2010), Identification of endogenous acyl amino acids based on a targeted lipidomics approach, J. Lipid Res. 51(1), 112-119)), as building blocks for antibiotics in bacterial cultures (Clardy, J., and Brady, S. F. (2007), Cyclic AMP directly activates NasP, an N-acyl amino acid antibiotic biosynthetic enzyme cloned from an uncultured beta-proteobacterium, J. Bacteriol. 189(17), 6487-6489) or as compounds involved in bacterial protein sorting (Craig, J. W., Cherry, M. A., Brady, S. F.(2011), Long-chain N-acyl amino acid synthases are linked to the putative PEP-CTERM/exosortase protein-sorting system in Gram-negative bacteria, J. Bacteriol. 193(20), 5707-5715).

Traditionally acyl amino acids have been produced at an industrial scale starting with materials derived from petrochemicals. More specifically, activated fatty acids provided in the form of acid chlorides may be used to acylate amino acids in an aqueous alkaline medium as described in GB 1 483 500. Shortcomings of such approaches include the need to add hazardous chemicals such as sulphuric acid or anhydrides thereof. Other synthetic approaches are associated with the accumulation of byproducts such as chloride salts which have undesirable effect on surfactancy.

A range of biotechnological routes towards production of acyl amino acids has been described. However, none of them is adequate for the commercial large-scale production of acyl amino acids owing to low yields, insufficient purities and the need for multi-step purification procedures. In particular only a small proportion of the carbon substrates fed to biotechnologically useful organisms is actually converted to the sought-after product, whilst much of it is consumed by reactions of the primary metabolism.

Another problem associated with biotechnological routes is the fact that a mixture of products is obtained the composition of which is difficult to control. More specifically, a range of fatty acids may be converted to acyl amino acids, even though production of but a single adduct may be desirable. Since the mixture comprises compounds highly related in terms of chemical structure, purifying or at least enriching a single component in an efficient and straightforward manner is usually beyond technical feasibility.

Therefore, the problem underlying the present invention is to provide an efficient biotechnological route towards acyl amino acids. In particular, the yield and purity of the product, in terms of catalysts or unwanted by-products, is to be improved compared to state of the art processes.

Another problem underlying the present invention is to provide a method for making acyl amino acids, wherein the spectrum of fatty acids converted to acyl amino acids is broader than is the case in state of the art processes. A particular objective is to provide a method suitable for converting short and unsaturated fatty acids to acyl amino acids.

Another problem underlying the present invention is to provide a biotechnological method for making acyl amino acids, wherein the length of the acyl residue in the acyl amino acid product may be controlled, preferably such that lauryl is enriched or prevalent.

The problem underlying the present invention is solved by the subject matter of the attached claims.

In a first aspect the problem underlying the present invention is solved by a cell expressing an amino acid-N-acyl-transferase, which is preferably recombinant, and an acyl-CoA synthetase, which is preferably recombinant,
wherein the cell has a reduced fatty acid degradation capacity.

In a first embodiment of the first aspect, the fatty acid degradation capacity of said cell is reduced owing to a decrease in activity, compared to the wild type cell, of at least one enzyme selected from the group comprising acyl-CoA dehydrogenase, 2,4-dienoyl-CoA reductase, enoyl-CoA hydratase and 3-ketoacyl-CoA thiolase, preferably acyl-CoA dehydrogenase.

In a second embodiment, which is also an embodiment of the first embodiment, wherein the amino acid-N-acyl-transferase is a human amino acid-N-acyl-transferase, preferably SEQ ID NO 4, SEQ ID NO 5 or a variant thereof.

In a third embodiment, which is also an embodiment of the first to second embodiment, the cell is a bacterial cell, preferably an enterobacterial cell, more preferably E. *coli.*

In a fourth embodiment, which is also an embodiment of the first to third embodiments, the cell is capable of making proteinogenic amino acids and/or fatty acids.

In a fifth embodiment, which is also an embodiment of the first to fourth embodiments, the cell expresses an acyl-CoA thioesterase, which is preferably recombinant and is more preferably SEQ ID NO 1 or a variant thereof.

In a sixth embodiment, which is also an embodiment of the first to fifth embodiments, the acyl-CoA synthetase is SEQ ID NO 6 or a variant thereof.

In a second aspect the problem underlying the present invention is solved by a method for producing acyl amino acids, comprising the steps
b) contacting an amino acid and an acyl CoA in the presence of an amino acid-N-acyl-transferase, which is preferably isolated and/or recombinant,
wherein the amino acid-N-acyl-transferase is preferably a human amino acid-N-acyl-transferase, more preferably SEQ ID NO 4, SEQ ID NO 5 or a variant thereof, and
wherein the acyl-CoA synthetase is preferably SEQ ID NO 6 or a variant thereof,
or culturing the cell according to first aspect or any embodiment thereof.

In a first embodiment of the second aspect, the method comprises, in addition to step b), the steps
a) converting a fatty acid to an acyl CoA using an acyl-CoA synthetase, which is preferably
   isolated and/or recombinant
   and/or
c) hydrogenating.

In a second embodiment, which is also an embodiment of the first embodiment, at least one of the enzymes, preferably all of them, selected from the group comprising amino acid-N-acyl-transferase and acyl-CoA synthetase is provided in the form of a cell expressing said enzyme or enzymes.

In a third embodiment, which is also an embodiment of the first or second embodiments, the cell expressing said enzyme or enzymes is the cell according to the first aspect or any embodiment thereof.

In a third aspect the problem underlying the present invention is solved by a reaction mixture comprising
an amino acid-N-acyl-transferase, which is preferably isolated and/or recombinant,
an acyl-CoA synthetase, which is preferably isolated and/or recombinant,
an amino acid and
either an acyl CoA or a fatty acid and an acyl-CoA-synthetase,
wherein the amino acid-N-acyl-transferase is preferably a human amino acid-N-acyl-transferase, more preferably SEQ ID NO 4, SEQ ID NO 5 or a variant thereof, and
wherein the acyl-CoA synthetase is preferably SEQ ID NO 6 or a variant thereof.

In a first embodiment of the third aspect, at least one of the enzymes, preferably all of them, selected from the group comprising an amino acid-N-acyl-transferase and an acyl-CoA synthetase is provided in the form of a cell, preferably the cell according to the first embodiment or any embodiment thereof.

In a third embodiment, which is also an embodiment of the first to second embodiments, the amino acid is a proteinogenic amino acid, preferably selected from the group comprising glycine, glutamine, glutamate, asparagine and alanine and is more preferably glycine.

In a fourth embodiment, which is also an embodiment of the first to third embodiments, the fatty acid is an unsaturated fatty acid and is preferably selected from the group comprising myristoleic acid, lauroleic acid, palmitoleic acid and cis-vaccenic acid.

In another embodiment of the second or third aspect, the fatty acid is a saturated fatty acid and is preferably selected from the group comprising laurate, myristate and palmitate.

In another embodiment of the second or third aspect, the fatty acid is provided in the form of an organic phase comprising a liquid organic solvent and the fatty acid, wherein the organic solvent is preferably an ester of the fatty acid.

In a fourth aspect, the problem underlying the present invention is solved by a composition comprising
a first acyl amino acid consisting of a saturated acyl having 8 to 16 carbon atoms and glycine,
a second acyl amino acid consisting of an unsaturated acyl having 10 to 18 carbon atoms and glycine,
and optionally a third acyl amino acid consisting of a saturated or unsaturated acyl having 12 carbon atoms and an amino acid selected from the group comprising glutamine, glutamic acid, alanine and asparagine.

In a first embodiment of the fourth aspect, the first acyl amino acid consists of a saturated acyl having 12 carbon atoms, preferably lauryl, and glycine.

In a second embodiment of the fourth aspect, which is also an embodiment of the first embodiment, the second acyl amino acid consists of an unsaturated acyl having 12 or 14 carbon atoms and glycine.

The present invention is based on the surprising finding that the combination of amino acid-N-acyl transferase and an acyl-CoA synthetase, preferably expressed by a cell having reduced fatty acid degradation capacity, may be used to convert a variety of fatty acids, more preferably a mixture comprising unsaturated and saturated fatty acids, to acyl amino acids.

Moreover, the present invention is based on the surprising finding that amino acid-N-acyl transferases exist that may be used to convert to an acyl amino acid short unsaturated fatty acids such as lauroleic acid.

Moreover, the present invention is based on the surprising finding that employing an amino acid-N-acyl-transferase capable of converting to an acyl amino acid a variety of fatty acids including short unsaturated fatty acids such as lauroleic acid may increase the yields of acyl amino acids produced.

Moreover, the present invention is based on the surprising finding that the composition of acyl amino acids produced in a cell, more specifically the length of fatty acids incorporated into such acyl amino acids, may be controlled by introducing into the cell one or more specific acyl-CoA thioesterases or altering the expression of one or more acyl-CoA thioesterases endogenously expressed by the cell.

The present invention centers around the use of an amino acid-N-acyl transferase and an acyl-CoA synthetase for making acyl amino acids. In a preferred embodiment, the term "amino acid-N-acyl transferase", as used herein, refers to an enzyme capable of catalyzing the conversion of acyl-CoA, preferably the CoA ester of lauroleic acid, and an amino acid, preferably a proteinogenic amino acid, more preferably glycine, to an acyl amino acid. Suitable amino acid-N-acyl transferases have been described in the prior art, for example in Waluk, D. P., Schultz, N., and Hunt, M. C. (2010), Identification of glycine N-acyltransferase-like 2 (GLYATL2) as a transferase that produces N-acyl glycines in humans, FASEB J. 24, 2795-2803. In a preferred embodiment, the amino acid-N-acyl transferase comprises a sequence selected from the group comprising SEQ ID NO 4, NP_001010904.1 and NP_659453.3 or a variant thereof. Throughout this application, any data base code, unless specified to the contrary, refers to a sequence available from the NCBI data bases, more specifically the version online on 5^{th} August 2013, and comprises, if such sequence is a nucleotide sequence, the polypeptide sequence obtained by translating the former.

The state of the art describes various methods to detect acyl-CoA synthetase activity. For example, the activity of an acyl-CoA synthetase may be assayed by incubating the sample of interest in 100 mM Tris-HCl at pH 8 in the presence of 250 µM lauroyl-CoA, 500 µM glycine and DTNB (5,5'-dithiobis-2-nitrobenzoic acid, also referred to as Ellman's reagent) and spectrophotometrically monitoring the absorbance at 410 nm following release of free thiol groups in the form of CoASH as the reaction progresses and reaction with Ellman's reagent.

In a preferred embodiment, the term "acyl amino acid", as used herein, refers to the product of the reaction catalysed by an amino acid-N-acyl transferase, more preferably a compound represented by the formula acyl-CO-NH-CHR-COOH, wherein R is the side chain of a proteinogenic amino acid, and wherein the term "acyl" refers to the acyl residue of a fatty acid. In a preferred embodiment of the present invention, the term "fatty acid", as used herein, means a carboxylic acid, preferably alkanoic acid, with at least 6, preferably 8, more preferably 10, most preferably 12 carbon atoms. In a preferred embodiment it is a linear fatty acid, in another embodiment it is branched. In a preferred embodiment it is a saturated fatty acid. In an especially preferred embodiment it is unsaturated. In another preferred embodiment it is a linear fatty acid with at least 12 carbon atoms comprising a double bond, preferably at position 9. In another preferred embodiment it is a simple unsaturated fatty acid having one double bond, which double bond is located at position 9 or 11. In the most preferred embodiment it is lauroleic acid (9-dodecenoic acid). In an especially preferred embodiment it is a fatty acid with 6, 7, 8, 9, 10, 11, 12, 13, 14, 15, 16, 17, 18, 19, 20, 21, 22, 23, 24, 25, 26, 27, 28, 29, 30 carbon atoms, preferably 12 carbon atoms.

Throughout this application, a formula referring to a chemical group that represents the dissociated or undissociated state of a compound capable of dissociating in an aqueous solution, comprises both the dissociated and the undissociated state and the various salt forms of the group. For example, the residue -COOH comprises both the protonated (-COOH) as well as the unprotonated (-COO⁻) carboxylic acid.

The acyl-CoA substrate consumed in the inventive reaction may be purified from a cell, chemically synthesised or produced using an acyl-CoA synthetase, the latter being the preferred option. In a preferred embodiment, the term "acyl-CoA synthetase", as used herein, refers to an enzyme capable of catalysing the ATP-dependent conversion of a fatty acid and CoA to acyl CoA. Various acyl-CoA synthetases have been described in the state of the art, for example YP_001724804.1, WP_001563489.1 and NP_707317.1. In a preferred embodiment, the acyl-CoA synthetase comprises SEQ ID NO 6 or YP_001724804.1 or a variant thereof. The activity of an acyl-CoA synthetase may be assayed as described in the state of the art, for example Kang, Y., Zarzycki-Siek, J., Walton, C. B., Norris, M. H., and Hoang, T. T. (2010), Multiple FadD Acyl-CoA Synthetases Contribute to Differential Fatty Acid Degradation and Virulence in Pseudomonas aeruginosa, PLOS ONE 5 (10), e13557. Briefly, the amount of free thiol in the form of unreacted CoASH is determined by adding Ellmann's reagent and spectrophotometrically monitoring the absorbance at 410 nm, preferably in a reaction buffer comprising 150 mM Tris-HCl (pH 7.2), 10 mM MgCl₂, 2 mM EDTA, 0.1 % Triton X-100, 5 mM ATP, 0.5 mM coenzyme A (CoASH) and a fatty acid (30 to 300 mM).

The teachings of the present invention may not only be carried out using biological macromolecules having the exact amino acid or nucleic acid sequences referred to in this application explicitly, for example by name or accession number, or implicitly, but also using variants of such sequences. In a preferred embodiment, the term "variant", as used herein, comprises amino acid or nucleic acid sequences, respectively, that are at least 70, 75, 80, 85, 90, 92, 94, 95, 96, 97, 98 or 99 % identical to the reference amino acid or nucleic acid sequence, wherein preferably amino acids other than those essential for the function, for example the catalytic activity of a protein, or the fold or structure of a molecule are deleted, substituted or replaced by insertions or essential amino acids are replaced in a conservative manner to the effect that the biological activity of the reference sequence or a molecule derived therefrom is preserved. The state of the art comprises algorithms that may be used to align two given nucleic acid or amino acid sequences and to calculate the degree of identity, see Arthur Lesk (2008), Introduction to bioinformatics, 3rd edition, Thompson et al., Nucleic Acids Research 22, 4637-4680, 1994, and Katoh et al., Genome Information, 16(1), 22-33, 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In a preferred embodiment, the term "variant", with regard to amino acid sequence, comprises, preferably in addition to the above sequence identity, amino acid sequences that comprise one or more conservative amino acid changes with respect to the respective reference or wild type sequence or comprises nucleic acid sequences encoding amino acid sequences that comprise one or more conservative amino acid changes. In a preferred embodiment, the term "variant" of an amino acid sequence or nucleic acid sequence comprises, preferably in addition to the above degree of sequence identity, any active portion and/or fragment of the amino acid sequence or nucleic acid sequence, respectively, or any nucleic acid sequence encoding an active portion and/or fragment of an amino acid sequence. In a preferred embodiment, the term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity. For example an active portion and/or fragment of a protease is capable of hydrolysing peptide bonds in polypeptides. In a preferred embodiment, the term "retains at least some of its essential biological activity", as used herein, means that the amino acid sequence in question has a biological activity exceeding and distinct from the background activity and the kinetic parameters characterising said activity, more specifically k_{cat} and K_{M}, are preferably within 3, more preferably 2, most preferably one order of magnitude of the values displayed by the reference molecule with respect to a specific substrate. In a preferred embodiment, the term "variant" of a nucleic acid comprises nucleic acids the complementary strand of which hybridises, preferably under stringent conditions, to the reference or wild type nucleic acid. Stringency of hybridisation reactions is readily determinable by one of ordinary skilled in the art, and in generally is an empirical calculation dependent on probe length, washing temperature and salt concentration. In general longer probes require higher temperatures for proper annealing, while shorter probes need lower temperatures. Hybridisation generally depends on the ability of denatured DNA to reanneal to complementary strands are present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995), Current Protocols in Molecular Biology. John Wiley & Sons, Inc. Moreover, the person skilled take in the art may follow the instructions given in the manual "The DIG System Users Guide for Filter Hybridization", Boehringer Mannheim GmbH, Mannheim, Germany, 1993 and in Liebl et al. (International Journal of Systematic Bacteriology 41: 255-260 (1991) on how to identify DNA sequences by means of hybridisation. In a preferred embodiment, stringent conditions are applied for any hybridisation, i.e. hybridisation occurs only if the probe is 70 % or more identical to the target sequence. Probes having a lower degree of identity with respect to the target sequence may hybridise, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50 °C - 68 °C, approximately 52 °C - 68 °C, approximately 54 °C-68 °C, approximately 56 °C - 68 °C, approximately 58 °C - 68 °C, approximately 60 °C - 68 °C, approximately 62 °C - 68 °C, approximately 64 °C - 68 °C, approximately 66 °C - 68 °C. In a particularly preferred embodiment, the temperature is approximately 64 °C - 68 °C or approximately 66 °C - 68 °C. It is possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. In a preferred embodiment, the term "homologue" of a nucleic acid sequence, as used herein, refers to any nucleic acid sequence that encodes the same amino acid sequence as the reference nucleic acid sequence, in line with the degeneracy of the genetic code.

If one or more of the enzymes used to practise the present invention is provided in the form of a cell, it is preferred that the cell has a reduced fatty acid degradation capacity. In a preferred embodiment, the term "having a reduced fatty acid degradation capacity", as used in herein, means that the respective cell degrades fatty acids, preferably those taken up from the environment, at a lower rate than a comparable cell or wild type cell having normal fatty acid degradation capacity would under identical conditions. In a preferred embodiment, the fatty acid degradation of such a cell is lower on account of deletion, inhibition or inactivation of at least one gene encoding an enzyme involved in the β-oxidation pathway. In a preferred embodiment of the present invention, at least one enzyme involved in the β-oxidation pathway has lost, in order of increasing preference, 5, 10, 20, 40, 50, 75, 90 or 99 % activity relative to the activity of the same enzyme under comparable conditions in the respective wild type microorganism. The person skilled in the art is familiar with various techniques that may be used to delete a gene encoding an enzyme or reduce the activity of such an enzyme in a cell, for example by exposition of cells to radioactivity followed by accumulation or screening of the resulting mutants, site-directed introduction of point mutations or knock out of a chromosomally integrated gene encoding for an active enzyme, as described in Sambrook/Fritsch/Maniatis (1989). In addition, the transcriptional repressor FadR may be over expressed to the effect that expression of enzymes involved in the β-oxidation pathway is repressed (Fujita, Y., Matsuoka, H., and Hirooka, K. (2007) Mol. Microbiology 66(4), 829-839). In a preferred embodiment, the term "deletion of a gene", as used herein, means that the nucleic acid sequence encoding said gene is modified such that the expression of active polypeptide encoded by said gene is reduced. For example, the gene may be deleted by removing in-frame a part of the sequence comprising the sequence encoding for the catalytic active centre of the polypeptide. Alternatively, the ribosome binding site may be altered such that the ribosomes no longer translate the corresponding RNA. Moreover, the person skilled in the art is able to routinely measure the activity of enzymes expressed by living cells using standard essays as described in enzymology text books, for example Cornish-Bowden (1995), Fundamentals of Enzym Kinetics, Portland Press Limited, 1995.

Degradation of fatty acids is accomplished by a sequence of enzymatically catalysed reactions. First of all, fatty acids are taken up and translocated across the cell membrane via a transport/acyl-activation mechanism involving at least one outer membrane protein and one inner membrane-associated protein which has fatty acid-CoA ligase activity, referred to in the case of *E. coli* as FadL and FadD / FadK, respectively. Inside the cell, the fatty acid to be degraded is subjected to enzymes catalyzing other reactions of the β-oxidation pathway. The first intracellular step involves the conversion of acyl-CoA to enoyl-CoA through acyl-CoA dehydrogenase, the latter referred to as FadE in the case of *E. coli.* The activity of an acyl-CoA dehydrogenase may be assayed as described in the state of art, for example by monitoring the concentration of NADH spectrophotometrically at 340 nm in 100 mM MOPS, pH 7.4, 0.2 mM Enoyl-CoA, 0.4 mM NAD⁺. The resulting enoyl-CoA is converted to 3-ketoacyl-CoA *via 3-*hydroxylacyl-CoA through hydration and oxidation, catalysed by enoyl-CoA hydratase/3-hydroxyacyl-CoA dehydrogenase, referred to as FadB and FadJ in *E. coli.* Enoyl-CoA hydratase/3-hydroxyacyl-CoA dehydrogenase activity, more specifically formation of the product NADH may be assayed spectrophotometrically as described in the state of the art, for example as outlined for FadE. Finally, 3-ketoacyl-CoA thiolase, FadA and Fadl in *E. coli,* catalyses the cleaveage of 3-ketoacyl-CoA, to give acetyl-CoA and the input acyl-CoA shortened by two carbon atoms. The activity of ketoacyl-CoA thiolase may be assayed as described in the state of the art, for example in Antonenkov, V., D.. Van Veldhoven, P., P., Waelkens, E., and Mannaerts, G.P. (1997) Substrate specificities of 3-oxoacyl-CoA thiolaseand sterol carrier protein 2/3-oxoacyl-coa thiolase purified from normal rat liver peroxisomes. Sterol carrier protein 2/3-oxoacyl-CoA thiolase is involved in the metabolism of 2-methyl-branched fatty acids and bile acid intermediates. J. Biol. Chem. 1997, 272:26023-26031*.* In a preferred embodiment, the term "a cell having a reduced fatty acid degradation capacity", as used herein, refers to a cell having a reduced capability of taking up and/or degrading fatty acids, preferably those having at least eight carbon chains. The fatty acid degradation capacity of a cell may be reduced in various ways. In a preferred embodiment, the cell has, compared to its wild type, a reduced activity of an enzyme involved in the β-oxidation pathway. In a preferred embodiment, the term "enzyme involved in the β-oxidation pathway", as used herein, refers to an enzyme that interacts directly with a fatty acid or a derivative thereof formed as part of the degradation of said fatty acid *via* the β-oxidation pathway the sequence of reactions effecting the conversion of a fatty acid to acetyl-CoA and the CoA ester of the shortened fatty acid, preferably by recognizing the fatty acid or derivative thereof as a substrate, and converts it to a metabolite formed as a part of the β-oxidation pathway. For example, the acyl-CoA dehydrogenase is an enzyme involved in the β-oxidation pathway as it interacts with fatty acid-CoA and converts fatty acid-CoA ester to enoyl-CoA, which is a metabolite formed as part of the β-oxidation. In a preferred embodiment, the term "enzyme involved in the β-oxidation pathway", as used herein, comprises any polypeptide from the group comprising acyl-CoA dehydrogenase, enoyl-CoA hydratase, 3-hydroxyacyl-CoA dehydrogenase and 3-keto-acyl-CoA thiolase. Subsequently, the acyl-CoA synthetase may catalyse the conversion a fatty acid to the CoA ester of a fatty acid, i.e. a molecule, wherein the functional group -OH of the carboxy group is replaced with -S-CoA, preferably for introducing said fatty acid into the β-oxidation pathway. For example, the polypeptides FadD and FadK in *E*. *coli* (access code: BAA15609.1) are acyl-CoA dehydrogenases. In a preferred embodiment, the term "acyl-CoA dehydrogenase", as used herein, is a polypeptide capable of catalysing the conversion of an acyl-CoA to enoyl-CoA, preferably as part of the β-oxidation pathway. For example, the polypeptide FadE in *E. coli* (access code: BAA77891.2) is an acyl-CoA dehydrogenase. In a preferred embodiment, the term "2,4-dienoyl-CoA reductase", as used herein, is a polypeptide capable of catalysing the conversion of the 2,4-dienoyl CoA from an unsaturated fatty acid into enoyl-CoA, preferably as part of the β-oxidation pathway. For example, the polypeptide FadH in *E*. *coli* is a 2,4-dienoyl-CoA reductase. In a preferred embodiment, the term "enoyl-CoA hydratase", as used herein, also referred to as 3-hydroxyacyl-CoA dehydrogenase, refers to a polypeptide capable of catalysing the conversion of enoyl-CoA to 3-ketoacyl-CoA through hydration and oxidation, preferably as part of the β-oxidation pathway. For example, the polypeptides FadB and FadJ in *E*. *coli* (access code: BAE77457.1) are enoyl-CoA hydratases. In a preferred embodiment, the term "ketoacyl-CoA thiolase", as used herein, refers to a polypeptide capable of catalysing the conversion of cleaving 3-ketoacyl-CoA, resulting in an acyl-CoA shortened by two carbon atoms and acetyl-CoA, preferably as the final step of the β-oxidation pathway. For example, the polypeptides FadA and Fadl in *E*. *coli* (access code: AP009048.1) are ketoacyl-CoA thiolases.

The inventive teachings may be carried out using a wide range of cells. In a preferred embodiment, the term "cell", as used herein, refers to any permanently unicellular organism comprising bacteria archaea, fungi, algae and the like. In a preferred embodiment, the cell is a bacterial cell, more preferably one from the group comprising *Pseudomonas, Corynebacterium, Bacillus* and *Escherichia,* most preferably *Escherichia coli.* In another preferred embodiment, the cell is a lower eukaryote, more preferably a fungus from the group comprising *Saccharomyces, Candida, Pichia, Schizosaccharomyces* and *Yarrowia,* and is most preferably *Saccharomyces cerevisiae.* The microorganism may be an isolated cell, in other words a pure culture of a single strain, or may comprise a mixture of at least two strains. Biotechnologically relevant cells are commercially available, for example from the American Type Culture Collection (ATCC) or the German Collection of Microorganisms and Cell Cultures (DSMZ). Particles for keeping and modifying cells are available from the prior art, for example Sambrook/Fritsch/Maniatis (1989): Molecular cloning - A Laboratory Manual, Cold Spring Harbour Press, 2nd edition, Fuchs/Schlegel (2007*),* Allgemeine Mikrobiologie, 2008, Georg Thieme Verlag*.*

Although the inventive teachings may be practiced using wild type cells, it is preferred that at least one of the enzymes involved, in particular at least one or all from the group comprising amino acid amino acid N-acyl-transferase, acyl-CoA synthetase and acyl-CoA thioesterase, is recombinant. In a preferred embodiment, the term "recombinant" as used herein, refers to a molecule or is encoded by such a molecule, preferably a polypeptide or nucleic acid, that, as such, does not occur naturally but is the result of genetic engineering or refers to a cell that comprises a recombinant molecule. For example, a nucleic acid molecule is recombinant if it comprises a promoter functionally linked to a sequence encoding a catalytically active polypeptide and the promoter has been engineered such that the catalytically active polypeptide is overexpressed relative to the level of the polypeptide in the corresponding wild type cell that comprises the original unaltered nucleic acid molecule.

Whether or not a nucleic acid molecule, polypeptide, more specifically an enzyme required to practice the invention, is recombinant or not has not necessarily implications for the level of its expression. However, it is preferred that one or more recombinant nucleic acid molecules, polypeptides or enzymes required to practice the invention are overexpressed. In a preferred embodiment, the term "overexpressed", as used herein, means that the respective polypeptide encoded or expressed is expressed at a level higher or at higher activity than would normally be found in the cell under identical conditions in the absence of genetic modifications carried out to increase the expression, for example in the respective wild type cell. The person skilled in the art is familiar with numerous ways to bring about overexpression. For example, the nucleic acid molecule to be overexpressed or encoding the polypeptide or enzyme to be overexpressed may be placed under the control of a strong inducible promoter such as the lac promoter. The state of the art describes standard plasmids that may be used for this purpose, for example the pET system of vectors exemplified by pET-3a (commercially available from Novagen). Whether or not a nucleic acid or polypeptide is overexpressed may be determined by way of quantitative PCR reaction in the case of a nucleic acid molecule, SDS polyacrylamide electrophoreses, Western blotting or comparative activity assays in the case of a polypeptide.

Besides, any of the enzymes required to practice the inventive teachings, in particular at least one or all from the group comprising N-acyl-transferase, acyl-CoA synthetase and acyl-CoA thioesterase, may be an isolated enzyme. In any event, any enzyme required to practice the present invention is preferably used in an active state and in the presence of all cofactors, substrates, auxiliary and/or activating polypeptides or factors essential for its activity. In a preferred embodiment, the term "isolated", as used herein, means that the enzyme of interest is enriched compared to the cell in which it occurs naturally. Whether or not an enzyme is enriched may be determined by SDS polyacrylamide electrophoresis and/or activity assays. For example, the enzyme of interest may constitute more than 5, 10, 20, 50, 75, 80, 85, 90, 95 or 99 percent of all the polypeptides present in the preparation as judged by visual inspection of a polyacrylamide gel following staining with Coomassie blue dye.

If a cell expressing an amino acid-N-acyl-transferase and an acyl-CoA synthetase and having a reduced fatty acid degradation capacity is used, it is preferred that the cell be capable of making proteinogenic amino acids and/or fatty acids. In a preferred embodiment, the term "proteinogenic amino acid", as used herein, refers to an amino acid selected from the group comprising alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine. Proteinogenic amino acids and fatty acids are synthesized as part of the primary metabolism of many wild type cells in reactions and using enzymes that have been described in detail in biochemistry textbooks, for example Jeremy M Berg, John L Tymoczko, and Lubert Stryer, Biochemistry, 5th edition, W. H. Freeman, 2002.

In a preferred embodiment, the cell used to practice the inventive teachings expresses an acyl-CoA thioesterase. In a more preferred embodiment, the term "acyl-CoA thioesterase", as used herein, refers to an enzyme capable of hydrolyzing acyl-CoA. In a preferred embodiment the acyl-CoA thioesterase comprises a sequence from the group comprising SEQ ID NO 1, AEM72521.1 and AAC49180.1 or a variant thereof, more preferably SEQ ID NO 1 or a variant thereof. The activity of acyl-CoA thioesterase may be assayed using various assays described in the state of the art. Briefly, the reaction of Ellman's reagent, which reacts with free thiol groups associated with CoASH formed upon hydrolysis of acyl-CoA may be detected by spectophotometrically monitoring absorbance at 412 nm.

In a preferred embodiment, the term "contacting", as used herein, means bringing about direct contact between the amino acid, the acyl CoA and the amino acid-N-acyl transferase or the inventive cell and/or any other reagents required to carry out the inventive teachings, preferably in an aqueous solution. For example, the cell, the amino acid and the acyl CoA may not be in different compartments separated by a barrier such as an inorganic membrane. If the amino acid or fatty acid is soluble and may be taken up by the cell or can diffuse across biological membranes, it may simply be added to the inventive cell in an aqueous solution. In case it is insufficiently soluble, it may be solved in a suitable organic solvent prior to addition to the aqueous solution. The person skilled in the art is able to prepare aqueous solutions of amino acids or fatty acids having insufficient solubility by adding suitable organic and/or polar solvents. Such solvents are preferably provided in the form of an organic phase comprising liquid organic solvent. In a preferred embodiment, the organic solvent or phase is considered liquid when liquid at 25 °C and standard atmospheric pressure. In another preferred embodiment, a fatty acid is provided in the form of a fatty acid ester such as the respective methyl or ethyl ester. For example, the fatty acid laurate may be solved in lauric acid methyl ester as described in EP11191520.3. According to the present invention, the compounds and catalysts may be contacted in vitro, i. e. in a more or less enriched or even purified state, or may be contacted in situ, i. e. they are made as part of the metabolism of the cell and subsequently react inside the cell.

The term "an aqueous solution" comprises any solution comprising water, preferably mainly water as solvent, that may be used to keep the inventive cell, at least temporarily, in a metabolically active and/or viable state and comprises, if such is necessary, any additional substrates. The person skilled in the art is familiar with the preparation of numerous aqueous solutions, usually referred to as media, that may be used to keep inventive cells, for example LB medium in the case of *E. coli.* It is advantageous to use as an aqueous solution a minimal medium, i.e. a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium.

It is a particular strength of the present invention that not only saturated fatty acids, but also unsaturated fatty acids may be converted to acyl amino acids. In case the end product sought-after is to comprise a higher yield of saturated acyl residues than is present after step b), it may be possible to complement the process by hydrogenating the acyl residues of the acyl amino acids following step b). In this case, the inventive composition according to the fifth aspect of the present invention, which composition comprises a mixture of acyl amino acids having unsaturated acyl residues, constitutes an obligatory intermediate which may be converted to the final product, i. e. a mixture of acyl amino acids having saturated acyl residues. The hydrogenation may be carried out according to various state of the art processes, for example those described in US5734070. Briefly, the compound to be hydrogenated may be incubated at 100 °C in the presence of hydrogen and a suitable catalyst, for example a nickel catalyst on silicon oxide as a support.

The inventions is further illustrated by the following figures and non-limiting examples from which further embodiments, aspects and advantages of the present invention may be taken.
**Fig. 1** depicts a total ion chromatogram of the 48 h sample from the *E. coli* W3110 ΔfadE pJ294{Ptac}[synUcTE] / pCDF{Ptac}[hGLYAT2(co_Ec)_fadD_Ec] fermentation.
**Fig. 2** depicts a total ion chromatogram of the 48 h sample from the *E. coli* W3110 ΔfadE pJ294{Ptac}[synUcTE] / pCDF{Ptac}[hGLYAT3(co_Ec)_fadD_Ec] fermentation.
**Fig. 3** depicts a total ion chromatogram of the 48 h sample from the *E. coli* W3110 ΔfadE pJ294{Ptac}[synUcTE] / pCDFDuet-1 fermentation (negative control).

### Examples

### Sequence ID NOs:

Throughout this application a range of SEQ ID NOs are used which include:

| SEO ID NO: | Comment |
|---|---|
| 1 | *Umbellularia californica synUcTE* (an acyl-CoA thioesterase) gene (codon-optimized) |
| 2 | tac promoter |
| 3 | Vector pJ294[Ptac-synUcTE], see example 1 |
| 4 | *Homo sapiens* genes hGLYAT2 (an amino acid N-acyl transferase) |
| 5 | *Homo sapiens* genes hGLYAT3 (another amino acid N-acyl transferase) |
| 6 | *Escherichia coli fadD* (an acyl-CoA synthetase) |
| 7 | Vector pCDF[atfA1_Ab(co_Ec)-fadD_Ec], see example 2 |
| 8 | Vector pCDF{Ptac}[hGLYAT2(co_Ec)-fadD_Ec], see example 2 |
| 9 | Vector pCDF{Ptac}[hGLYAT3(co_Ec)-fadD_Ec], see example 2 |
| 10 | alkL (an importer facilitating transport of hydrophobic acyl across cell membranes) gene, see example 3 |
| 11 | lacuv5 promoter, see example 3 |
| 12 | Vector pCDF[alkLmod1], see example 3 |
| 13 | Vector pCDF{Ptac}[hGLYAT2(co_Ec)-fadD_Ec]{Placuv5}[alkLmod1], see example 3 |

### Example 1

### Generation of an expression vector for the Umbellularia californica gene synUcTE

To generate an expression vector for the *Umbellularia californica synUcTE* gene (SEQ ID No. 1), which encodes the *Umbellularia californica* acyl CoA-thioesterase, this gene was codon-optimized for expression in *Escherichia coli.* The gene was synthesized together with a *tac* promoter (SEQ ID No. 2), and, simultaneously, one cleavage site was introduced upstream of the promoter and one cleavage site downstream of the terminator. The synthesized DNA fragment P_{tac}-synUcTE was digested with the restriction endonucleases *BamH*I and Notl and ligated into the correspondingly cut vector pJ294 (DNA2.0 Inc., Menlo Park, CA, USA). The finished *E. coli* expression vector was referred to as pJ294[Ptac-synUcTE] (SEQ ID No. 3).

### Example 2

### Generation of vectors for coexpression of Escherichia coli fadD with either the Homo sapiens genes hGLYAT3 and hGLYAT2

To generate vectors for the coexpression of the *Homo sapiens* genes hGLYAT2 (SEQ ID No. 4) or hGYLAT3 (SEQ ID No. 5), which encodes human glycine-N-acyltransferase, with *Escherichia coli fadD* (SEQ ID No. 6), which encodes the *E. coli* acyl-CoA synthetase, the genes hGLYAT2 and hGLYAT3 were codon-optimized for expression in *Escherichia coli* and synthesized. The synthesized DNA fragments were digested with the restriction endonucleases *Sac*II and *Eco*47III and ligated into the correspondingly cut pCDF[atfA1_Ab(co_Ec)-fadD_Ec] (SEQ ID No. 7) with removal of the *aftA*1 gene. The sequence segments which were additionally removed in this process were cosynthesized during gene synthesis. The vector is a pCDF derivative which already comprises a synthetic tac promoter (SEQ ID No. 2) and the *Escherichia coli fadD* gene. The resulting expression vectors were named pCDF{Ptac}[hGLYAT2(co_Ec)-fadD_Ec] (SEQ ID No. 8) and pCDF{Ptac}[hGLYAT3(co_Ec)-fadD_Ec] (SEQ ID No. 9).

### Example 3

### Generation of vectors for the coexpression of the Homo sapiens hGLYAT2, Escherichia coli fadD and Pseudomonas putida alkL genes

To generate vectors for the coexpression of the hGLYAT2 genes with a modified *Pseudomonas putida* alkL gene, which encodes AlkL, an outer membrane protein that facilitates the import of hydrophobic substrates into a cell, the alkL gene (SEQ ID No. 10) was amplified together with the lacuv5 promoter (SEQ ID No. 11) from the plasmid pCDF[alkLmod1] (SEQ ID No. 12) by means of sequence-specific oligonucleotides. The PCR products were cleaved with the restriction endonucleases *Bam*HI and *Nsil* and ligated into the correspondingly cleaved vector pCDF{Ptac}[hGLYAT2(co_Ec)-fadD_Ec] (SEQ ID No. 8). The correct insertion of the target genes was checked by restriction analysis and the authenticity of the introduced genes was verified by DNA sequencing. The resulting expression vector was named pCDF{Ptac}[hGLYAT2(co_Ec)-fadD_Ec]{Placuv5}[alkLmod1] (SEQ ID No. 13).

### Example 4

### Generation of an E. coli strain with deletion in the fadE gene, which strain overexpresses the Umbellularia californica synUcTE, Escherichia coli fadD and Homo sapiens hGLYAT2 and hGLYAT3 genes

To generate *E*. *coli* strains which coexpress the *Umbellularia californica synUcTE* in combination with the *Escherichia coli fadD* and *Homo sapiens* hGLYAT2 or *Homo sapiens* hGLYAT3 genes, the strain *E.coli* W3110 ΔfadE was transformed with the plasmids pJ294{Ptac}[synUcTE] and pCDF{Ptac}[hGLYAT2(co_Ec)_fadD_Ec] or pCDF{Ptac}[hGLYAT3(co_Ec)_fadD_Ec] by means of electroporation and plated onto LB-agar plates supplemented with spectinomycin (100 µg/ml) and ampicillin (100 µg/ml). Transformants were checked for the presence of the correct plasmids by plasmid preparation and analytic restriction analysis. The strains *E.coli* W3110 ΔfadE pJ294{Ptac}[synUcTE] / pCDF{Ptac}[hGLYAT2(co_Ec)_fadD_Ec] and *E.coli* W3110 ΔfadE pJ294{Ptac}[synUcTE] / pCDF{Ptac}[hGLYAT3(co_Ec)_fadD_Ec] were generated thus.

### Example 5

### Generation of an E.coli strain with deletion in the fadE gene, which strain overexpresses the Escherichia coli fadD and either Homo sapiens hGLYAT2 or hGLYAT3 genes

To generate *E*. *coli* strains which overexpress the *Escherichia coli fadD* gene in combination with the *Homo sapiens* hGLYAT2 or hGLYAT3 genes, electrocompetent cells of *E*. *coli* strain W3110 ΔfadE were generated. *E.coli* W3110 ΔfadE was transformed with the plasmid pCDF{Ptac}[hGLYAT2(co_Ec)-fadD_Ec]{Placuv5}[alkLmod1] and plated onto LB-agar plates supplemented with spectinomycin (100 µg/ml). Transformants were checked for the presence of the correct plasmids by plasmid preparation and analytic restriction analysis. The strain generated thus was named *E*. *coli* W3110 ΔfadE pCDF{Ptac}[hGLYAT2(co_Ec)-fadD_Ec]{Placuv5}[alkLmod1].

### Example 6

### Production of fatty acid/amino acid adducts by E. coli strains with deletion in the fadE gene, which strains overexpress the synUcTEand fadD genes in combination with either hGLYAT2 or hGLYAT3

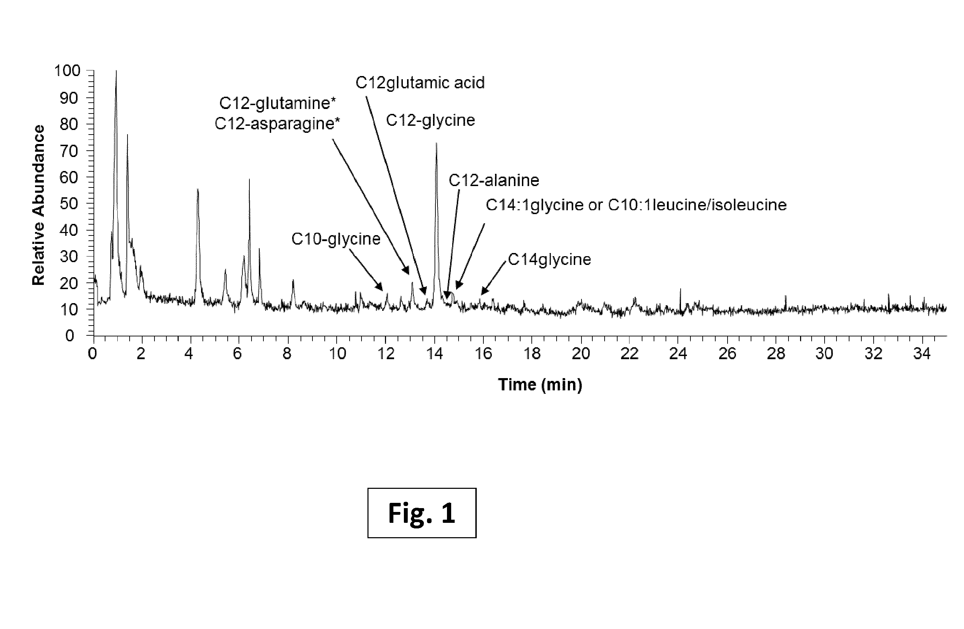

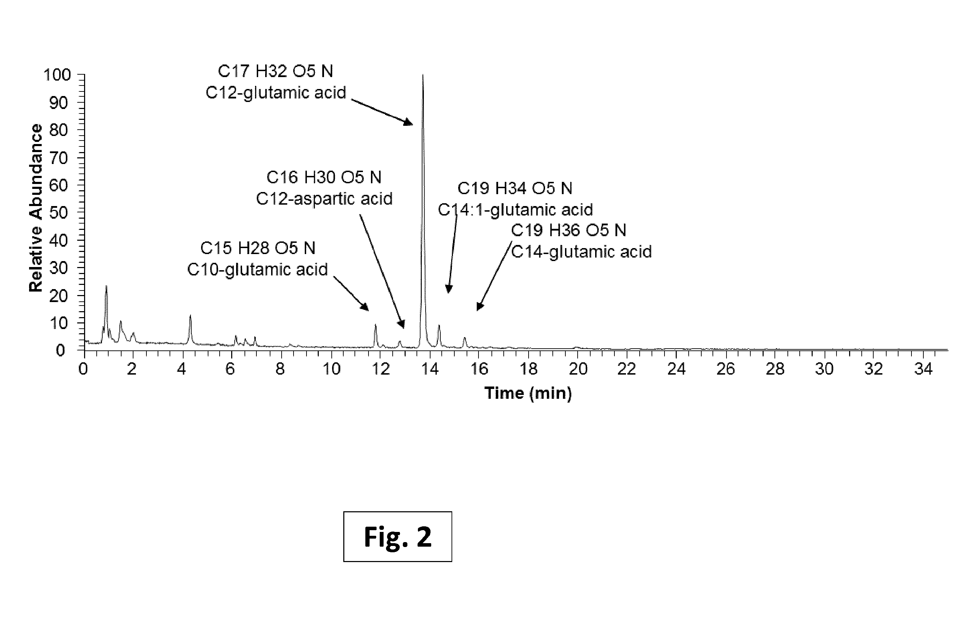

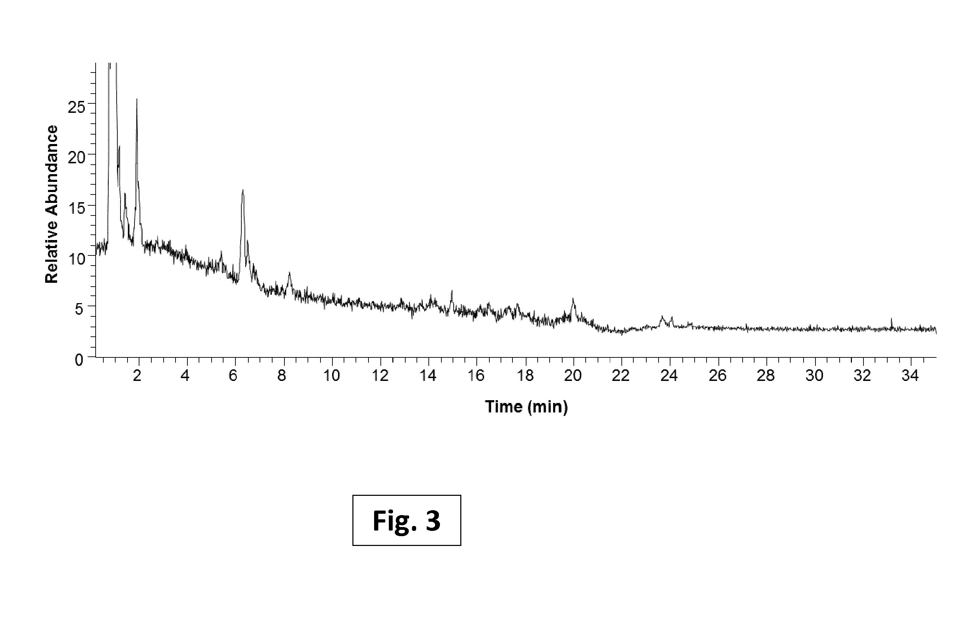

The strains generated in Example 4 were used to study their ability to produce fatty acid/amino acid adducts, proceeding as follows:
Starting from a -80 °C glycerol culture, the strains to be studied were first plated onto an LB-agar plate supplemented with 100 µg/ml ampicillin and 100 µg/ml spectinomycin and incubated overnight at 37 °C. Starting from a single colony in each case, the strains were then grown as a 5-ml preculture in Luria-Bertani broth, Miller (Merck, Darmstadt) supplemented with 100 µg/ml ampicillin and 100 µg/ml spectinomycin. The further culture steps were performed in M9 medium. The medium, composed of 38 mM disodium hydrogenphosphate dihydrate, 22 mM potassium dihydrogenphosphate, 8.6 mM sodium chloride, 37 mM ammonium chloride, 2 % (w/v) glucose, 2 mM magnesium sulphate heptahydrate (all chemicals from Merck, Darmstadt) and 0.1 % (v/v) trace element solution, was brought to pH 7.4 with 25 % strength ammonium hydroxide solution. The trace element solution added, composed of 9.7 mM manganese(II) chloride tetrahydrate, 6.5 mM zinc sulphate heptahydrate, 2.5 mM sodium-EDTA (Titriplex III), 4.9 mM boric acid, 1 mM sodium molybdate dihydrate, 32 mM calcium chloride dihydrate, 64 mM iron(II) sulphate heptahydrate and 0.9 mM copper(II) chloride dihydrate, dissolved in 1 M hydrochloric acid (all chemicals from Merck, Darmstadt) was filter-sterilized before being added to the M9 medium. 20 ml of M9 medium supplemented with 100 µg/ml spectinomycin and 100 µg/ml ampicillin were introduced into baffled 100-ml Erlenmeyer flasks and inoculated with 0.5 ml preculture. The flasks were cultured at 37 °C and 200 rpm in a shaker-incubator. After a culture time of 8 hours, 50 ml of M9 medium supplemented with 100 µg/ml spectinomycin and 100 µg/ml ampicillin were introduced into a baffled 250-ml Erlenmeyer flask and inoculated with the 10-ml culture to achieve an optical density (600 nm) of 0.2. The flasks were cultured at 37 °C and 200 rpm in a shaker-incubator. When an optical density (600 nm) of 0.7 to 0.8 was reached, gene expression was induced by addition of 1 mM IPTG. The strains were cultured for a further 48 hours at 30 °C and 200 rpm. Simultaneously with the induction, 1 g/l glycine was added to some of the cultures. During culturing, samples were taken, and fatty acid/amino acid adducts present were analysed. The results are shown in **Figs. 1** and **2****.** It has been possible to demonstrate that both *E.coli* strain W3110 ΔfadE pJ294{Ptac}[synUcTE] / pCDF{Ptac}[hGLYAT2(co_Ec)_fadD_Ec] and *E.coli* strain W3110 ΔfadE pJ294{Ptac}[synUcTE] / pCDF{Ptac}[hGLYAT3(co_Ec)_fadD_Ec] are capable of forming various fatty acid/amino acid adducts, for example lauroyl-glutamic acid, from glucose. By contrast, no such adducts can be found in a cell that, as a negative control, lacks the plasmids (Fig. 3). It appears that slight sequence variations, for example amino acid substitutions that distinguish hGLYAT2 from hGLYAT3, do not compromise the ability of the cell to make the fatty acid/acyl amino acid adducts.

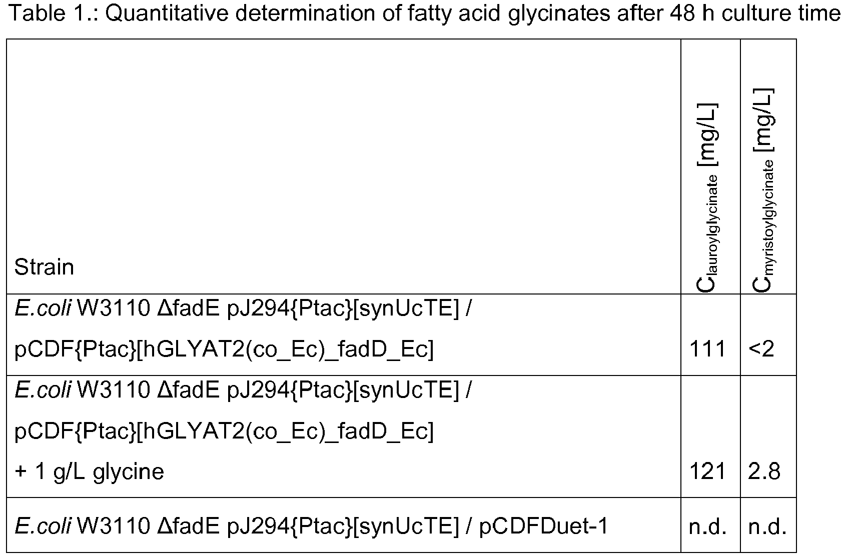

### Example 7

### Chromatographic quantification of products by HPLC/MS

The quantification of N-lauroylglycine, N-myristoylglycine and N-palmitoylglycine and the detection of other acylamino acids in fermentation samples was performed by HPLC-ESI/MS. The quantification was performed with the aid of an external calibration (approx. 0.1 - 50 mg/l) for the three target compounds in the "single ion monitoring" mode (SIM). In parallel, a scan was carried out over a mass range m/z = 100-1000 so as to identify further acylamino acids.

The samples for the determination of the fatty acid glycinates were prepared as follows: 800 µl of solvent (acetone) and 200 µl of sample were pipetted into a 2-ml reaction vessel. The mixture was shaken in a Retsch mill for 1 minute at 30 Hz and then centrifuged for 5 min at approximately 13 000 rpm. The clear supernatant was removed using a pipette and, after suitable dilution with diluent (80 % acetonitrile/20 % water + 0.1 % formic acid), analyzed. The calibration standards used were likewise dissolved and diluted in this diluent.

The following equipment was employed:
- Surveyor HPLC system (Thermo Scientific, Waltham, Massachusetts, USA) composed of MS pump, Autosampler Plus and PDA Detector Plus
- Mass spectrometer TSQ Vantage with HESI II source (Thermo Scientific, Waltham, Massachusetts, USA)
- HPLC column: 100 x 2 mm Pursuit XRS Ultra C8; 2.8 µm (Agilent, Santa Clara, California, USA)

Chemicals:
- Water from a Millipore system
- Acetonitrile for HPLC (Merck AG, Darmstadt, Germany)
- Formic acid, p.a. grade (Merck, Darmstadt, Germany)
- N-propanol Lichrosolv (Merck, Darmstadt, Germany)
- N-lauroylglycine 99 % (Chem-Impex International, Wood Dale, IL, USA)
- N-myristoylglycine > 98 % (Santa Cruz Biotechnology, Texas, USA)
- N-palmitoylglycine > 99 % (provenance unknown)

The HPLC separation was carried out using the abovementioned HPLC column. The injection volume amounted to 2 µl, the column temperature to 40 °C, the flow rate to 0.3 ml/min. The mobile phase consisted of Eluent A (0.1 % strength (v/v) aqueous formic acid) and Eluent B (75 % acetonitrile/25 % n-propanol (v/v) with 0.1 % (v/v) formic acid). The following gradient profile was used:

| Time [min] | Eluent A [%] | Eluent B [%] |
|---|---|---|
| 0 | 90 | 10 |
| 1 | 90 | 10 |
| 20 | 5 | 95 |
| 25 | 5 | 95 |

The HPLC/MS analysis was carried out under positive ionization mode with the following parameters of the ESI source:

| | |
|---|---|
| Spray Voltage: | 3500V |
| Vaporizer Temperature: | 50 °C |
| Sheath Gas Pressure: | 40 |
| Aux. Gas Pressure: | 10 |
| Capillary Temperature: | 250 °C |
| Sprayer Distance: | Ring C |

Detection and quantification of the three analytes were performed by "single ion monitoring" (SIM) with the following parameters:

| **Analyte** | **Ion [M+H][m/z]** | **Scan range [m/z]** | **Scan time [ms]** | **Resolution Q3** |
|---|---|---|---|---|
| N-lauroylglycine | 258.2 | 0.002 | 50 | 0.7 |
| N-myristoylglycine | 286.2 | 0.002 | 50 | 0.7 |
| N-palmitoylglycine | 314.2 | 0.002 | 50 | 0.7 |

### Example 8

### Production of fatty acid amino acid adducts by E.coli strains with deletion in the fadE gene, which strains overexpress the synUcTEand fadD genes in combination with hGLYAT2 or hGLYAT3 in a parallel fermentation system

The strains generated in Example 4 were used for studying their ability to produce fatty acid amino acid adducts from glucose. For this purpose, the strain was cultured both in a shake flask and in a fed-batch fermentation. The fermentation was carried out in a parallel fermentation system from DASGIP with 8 bioreactors.

The production cells were prepared as described in Example 6.

The fermentation was performed using 1 I reactors equipped with overhead stirrers and impeller blades. pH and pO₂ were measured online for process monitoring. OTR/CTR measurements served for estimating the metabolic activity and cell fitness, inter alia.

The pH electrodes were calibrated by means of a two-point calibration using standard solutions of pH 4.0 and pH 7.0, as specified in DASGIP's technical instructions. The reactors were provided with the necessary sensors and connections as specified in the technical instructions, and the agitator shaft was fitted. The reactors were then charged with 300 ml of water and autoclaved for 20 min at 121 °C to ensure sterility. The pO₂ electrodes were connected to the measuring amplifiers and polarized overnight (for at least 6 h). Thereafter, the water was removed under a clean bench and replaced by M9 medium (pH 7.4) composed of KH₂PO₄ 3.0 g/l, Na₂HPO₄ 6.79 g/l, NaCl 0.5 g/l, NH₄Cl 2.0 g/l, 2 ml of a sterile 1 M MgSO₄*7H₂O solution and 1 ml/I of a filter-sterilized trace element stock solution (composed of HCl (37 %) 36.50 g/l, MnCl₂*4H₂O 1.91 g/l, ZnSO₄*7H₂O 1.87 g/l, ethylenediaminetetraacetic acid dihydrate 0.84 g/l, H₃BO₃ 0.30 g/l, Na₂MoO₄*2H₂O 0.25 g/l, CaCl₂*2H₂O 4.70 g/l, FeSO₄*7H₂O 17.80 g/l, CuCl₂*2H₂O 0.15 g/l) with 15 g/l glucose as the carbon source (added by metering in 30 ml/I of a sterile feed solution composed of 500 g/l glucose, 1.3 % (w/v) MgSO₄*7H₂O) supplemented with 100 mg/l spectinomycin and 3 ml/l DOW1500.

Thereafter, the pO₂ electrodes were calibrated to 100 % with a one-point calibration (stirrer: 400 rpm/aeration: 10 sl/h air), and the feed, correction agent and induction agent lines were cleaned by "cleaning in place" as specified in the technical instructions. To this end, the tubes were rinsed first with 70 % ethanol, then with 1 M NaOH, then with sterile fully-demineralized water and, finally, filled with the respective media. Using the *E. coli* strain of Example 4, a dilution streak was first performed with a cryoculture on an LB agar plate supplemented with 100 mg/l spectinomycin, and the plate was incubated for approximately 16 h at 37 °C. LB medium (10 ml in a 100-ml baffle flask) supplemented with 100 mg/l spectinomycin was then inoculated with a single colony and the culture was grown overnight at 37 °C and 200 rpm for approximately 16 h. Thereafter, this culture was used for a second preculture stage with an initial OD of 0.2 in 50 ml of M9 medium, composed of KH₂PO₄ 3.0 g/l, Na₂HPO₄ 6.79 g/l, NaCl 0.5 g/l, NH₄Cl 2.0 g/l, 2 ml of a sterile 1 M MgSO₄*7H₂O solution and 1 ml/I of a filter-sterilized trace element stock solution (composed of HCl (37 %) 36.50 g/l, MnCl₂*4H₂O 1.91 g/l, ZnSO₄*7H₂O 1.87 g/l, ethylenediaminetetraacetic acid dihydrate 0.84 g/l, H₃BO₃ 0.30 g/l, Na₂MoO₄*2H₂O 0.25 g/l, CaCl₂*2H₂O 4.70 g/l, FeSO₄*7H₂O 17.80 g/l, CuCl₂*2H₂O 0.15 g/l) supplemented with 20 g/l glucose as carbon source (added by metering in 40 ml/I of a sterile feed solution composed of 500 g/l glucose) together with the above-described antibiotics was transferred into a 500-ml baffle flask and incubated for 8-12 h at 37 °C/200 rpm. To inoculate the reactors with an optical density of 0.1, the OD₆₀₀ of the second preculture stage was measured and the amount of culture required for the inoculation was calculated. The required amount of culture was placed into the heated and aerated reactor with the aid of a 5-ml syringe through a septum.

The following standard program was used:

| DO controller | | pH controller | |
|---|---|---|---|
| Preset | 0% | Preset | 0 ml/h |
| P | 0.1 | P | 5 |
| Ti | 300 s | Ti | 200 s |
| Min | 0% | Min | 0 ml/h |
| Max | 100 % | Max | 40 ml/h |

| N (Rotation) | from | to | XO2 (gas mixture) | from | to | F (gas flow) | from | to |
|---|---|---|---|---|---|---|---|---|
| Growth and biotransformation | 0% | 30% | Growth and biotransformation | 0% | 100 % | Growth and biotransformation | 15% | 80% |
| | 400 rpm | 1500 rpm | | 21 % | 21 % | | 6 sl/h | 72 sl/h |

| Script | |
|---|---|
| Trigger fires | 31 % DO (1/60h) |
| Induction IPTG | 2 h after the feed start |
| Feed trigger | 50 % DO |
| Feed rate | 3 [ml/h] |

The pH was adjusted unilaterally to pH 7.0 with 12.5 % strength ammonia solution. During the growth phase and the biotransformation, the dissolved oxygen (pO₂ or DO) in the culture was adjusted to at least 30 % via the stirrer speed and the aeration rate. After the inoculation, the DO dropped from 100 % to these 30 %, where it was maintained stably for the remainder of the fermentation.

The fermentation was carried out as a fed batch, the feed start as the beginning of the feed phase with 5 g/l*h glucose feed, composed of 500 g/l glucose, 1.3 % (w/v) MgSO₄*7H₂O, being triggered via the DO peak which indicates the end of the batch phase. From the feed start onwards, the temperature was reduced from 37 °C to 30 °C. 2 h after the feed start, the expression was induced with 1 mM IPTG.

To quantify lauroyl, myristoyl and palmitoyl glycinate, samples were taken 47 h and 64 h after the start of the fermentation. These samples were prepared for analysis, and analyzed as described in Example 7.

It has been possible to demonstrate that strain E. coli W3110 ΔfadE pJ294{Ptac}[synUcTE] / pCDF{Ptac}[hGLYAT2(co_Ec)_fadD_Ec] is capable of forming lauroyl glycinate from glucose.

**Table 2: Quantification of fatty acid glycinates after 47 and 64 h fermentation time.**

| Analyte | Ion [M+H] [m/z] | Scan range [m/z] | Scan time [ms] | Resolution Q3 |
|---|---|---|---|---|
| N-Lauroylglycine | 258.2 | 0.002 | 50 | 0.7 |
| N-Myristoylglycine | 286.2 | 0.002 | 50 | 0.7 |
| N-Palmitoylglycine | 314.2 | 0.002 | 50 | 0.7 |

Production of fatty acids after 47 and 64 hours' fermentation time. (n. d.: not determined)

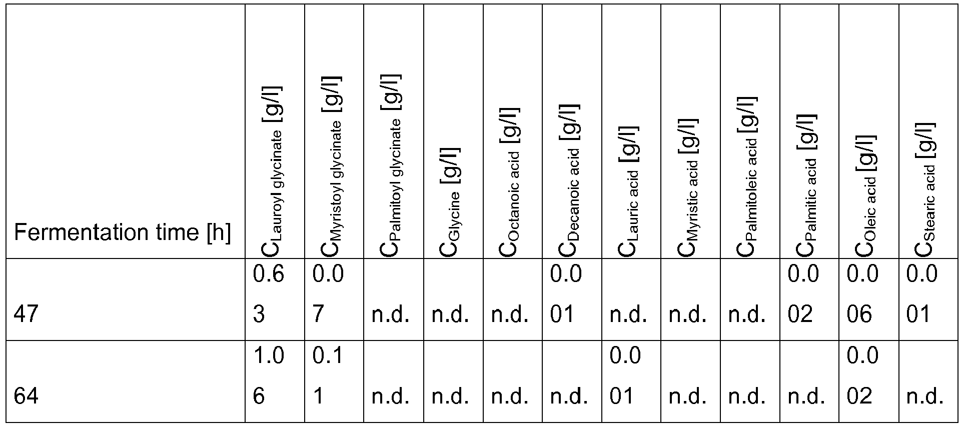

### Example 9

The strain of Example 5 was fermented in a fed-batch fermentation to study the ability of linking lauric acid and glycine to give lauroyl glycinate. This fermentation was carried out in a parallel fermentation system from DASGIP with 8 bioreactors.

The experimental setting was as described in Example 8 except that 100 g/l glycine in demineralized water and 100 g/l laurate in lauric acid methyl ester were fed rather than glucose.. To quantify lauroyl, myristoyl and palmitoyl glycinate in fermentation samples, samples were taken 23 h and 42 h after the start of the fermentation. These samples were prepared for analysis, and analyzed as described in Example 7.

It has been possible to demonstrate that the strain E.coli W3110 ΔfadE pCDF{Ptac}[hGLYAT2(co_Ec)_fadD_Ec] {Plavuv5} [alkLmod1] is capable of linking lauric acid and glycine and of producing lauroyl glycinate.

Production of lauroyl glycinate after fermentation for 23 and 42 hours with feeding of lauric acid and glycine.

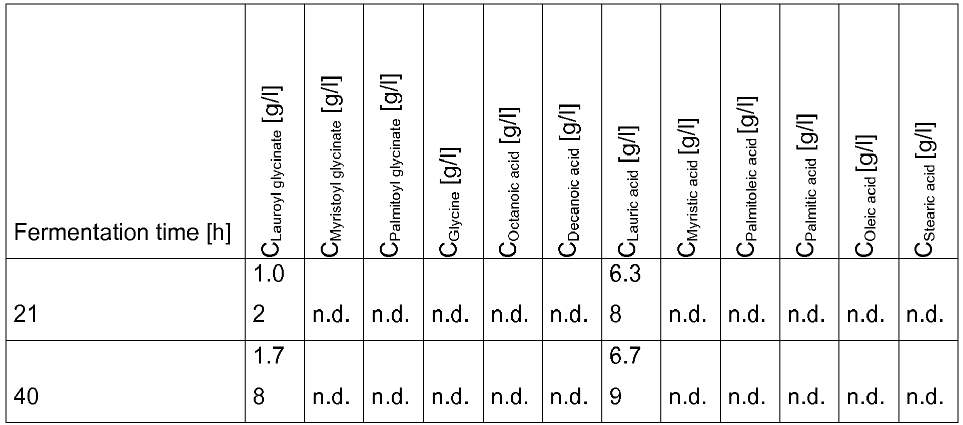

Production of lauroyl glycinate after fermentation for 23 and 42 hours without feeding of lauric acid and glycine (negative control).

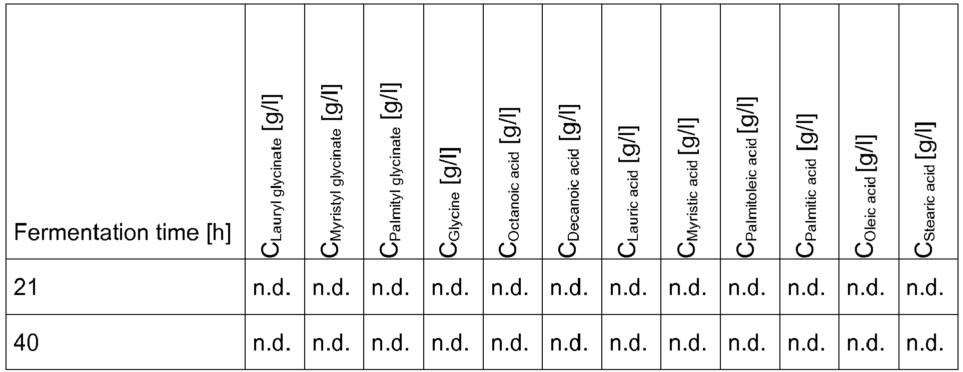

## Claims

1. A cell expressing an amino acid-N-acyl-transferase, which is preferably recombinant, and an acyl-CoA synthetase, which is preferably recombinant,
wherein the cell has a reduced fatty acid degradation capacity.

2. The cell according to claim 1, wherein the fatty acid degradation capacity is reduced owing to a decrease in activity, compared to the wild type cell, of at least one enzyme selected from the group comprising acyl-CoA dehydrogenase, 2,4-dienoyl-CoA reductase, enoyl-CoA hydratase and 3-ketoacyl-CoA thiolase, preferably acyl-CoA dehydrogenase.

3. The cell according to any of claims 1 to 2, wherein the amino acid-N-acyl-transferase is a human amino acid-N-acyl-transferase, preferably SEQ ID NO 4, SEQ ID NO 5 or a variant thereof.

4. The cell according to any of claims 1 to 3, wherein the cell is a bacterial cell, preferably an enterobacterial cell, more preferably E. *coli.*

5. The cell according to any of claims 1 to 4, wherein the cell is capable of making proteinogenic amino acids and/or fatty acids.

6. The cell according to any of claims 1 to 5, wherein the cell expresses an acyl-CoA thioesterase, which is preferably recombinant and is more preferably SEQ ID NO 1 or a variant thereof.

7. The cell according to any of claims 1 to 6, wherein the acyl-CoA synthetase is SEQ ID NO 6 or a variant thereof.

8. A method for producing acyl amino acids, comprising the steps
b) contacting an amino acid and an acyl CoA in the presence of an amino acid-N-acyl-transferase, which is preferably isolated and/or recombinant,
wherein the amino acid-N-acyl-transferase is preferably a human amino acid-N-acyl-transferase, more preferably SEQ ID NO 4, SEQ ID NO 5 or a variant thereof, and wherein the acyl-CoA synthetase is preferably SEQ ID NO 6 or a variant thereof.
or culturing the cell according to any of claims 1 to 7.

9. The method according to claim 8, further comprising, in addition to step b), the steps
a) converting a fatty acid to an acyl CoA using an acyl-CoA synthetase, which is preferably isolated and/or recombinant,
and/or
c) hydrogenating.

10. The method according to any of claims 8 to 9, wherein at least one of the enzymes, preferably all of them, selected from the group comprising amino acid-N-acyl-transferase and acyl-CoA synthetase is provided in the form of a cell expressing said enzyme or enzymes.

11. The method according to claim 10, wherein the cell expressing said enzyme or enzymes is the cell according to any of claims 1 to 7.

12. A reaction mixture comprising
an amino acid-N-acyl-transferase, which is preferably isolated and/or recombinant,
an acyl-CoA synthetase, which is preferably isolated and/or recombinant,
an amino acid and
either an acyl CoA or a fatty acid and an acyl-CoA-synthetase,
wherein the amino acid-N-acyl-transferase is preferably a human amino acid-N-acyl-transferase, more preferably SEQ ID NO 4, SEQ ID NO 5 or a variant thereof, and wherein the acyl-CoA synthetase is preferably SEQ ID NO 6 or a variant thereof.

13. The reaction mixture according to claim 12, wherein at least one of the enzymes, preferably all of them, selected from the group comprising an amino acid-N-acyl-transferase and an acyl-CoA synthetase is provided in the form of a cell, preferably the cell according to any of claims 1 to 7.

14. The method or reaction mixture according to any of claims 8 to 13, wherein the amino acid is a proteinogenic amino acid, preferably selected from the group comprising glycine, glutamine, glutamate, asparagine and alanine and is more preferably glycine.

15. The method or reaction mixture according to any of claims 8 to 14, wherein the fatty acid is an unsaturated fatty acid and is preferably selected from the group comprising myristoleic acid, lauroleic acid, palmitoleic acid and cis-vaccenic acid.

16. The method or reaction mixture according to any of claims 8 to 14, wherein the fatty acid is a saturated fatty acid and is preferably selected from the group comprising laurate, myristate and palmitate.

17. The method or reaction mixture according to any of claims 8 to 16, wherein the fatty acid is provided in the form of an organic phase comprising a liquid organic solvent and the fatty acid, wherein the organic solvent is preferably an ester of the fatty acid.

18. A composition comprising
a first acyl amino acid consisting of a saturated acyl having 8 to 16 carbon atoms and glycine,
a second acyl amino acid consisting of an unsaturated acyl having 10 to 18 carbon atoms and glycine,
and optionally a third acyl amino acid consisting of a saturated or unsaturated acyl having 12 carbon atoms and an amino acid selected from the group comprising glutamine, glutamic acid, alanine and asparagine.

19. The composition according to claim 18, wherein the first acyl amino acid consists of a saturated acyl having 12 carbon atoms, preferably lauryl, and glycine.

20. The composition according to any of claims 18 to 19, wherein the second acyl amino acid consists of an unsaturated acyl having 12 or 14 carbon atoms and glycine.
